Europäisches Patentamt

European Patent Office

Office européen des brevets

Veröffentlichungsnummer: **0 272 438**
**A1**

# EUROPÄISCHE PATENTANMELDUNG

Anmeldenummer: 87116700.3

Int. Cl.⁴ **A61K 7/06** , A61K 7 00

Anmeldetag: **12.11.87**

Priorität: **19.12.86 DE 3643418**

Veröffentlichungstag der Anmeldung:
**29.06.88 Patentblatt 88/26**

Benannte Vertragsstaaten:
**AT BE CH DE ES FR GB GR IT LI NL SE**

Anmelder: **Wella Aktiengesellschaft**
**Berliner Allee 65**
**D-6100 Darmstadt(DE)**

Erfinder: **Konrad, Eugen**
**Mecklenburger Strasse 101**
**D-6100 Darmstadt(DE)**
Erfinder: **Mager, Herbert, Dr.**
**Beaumont 5**
**CH-1700 Fribourg(CH)**
Erfinder: **Braun, Hans-Jürgen, Dr.**
**Im Dorf 32**
**CH-3183 Albligen(CH)**
Erfinder: **Clausen, Thomas, Dr.**
**Ernst Pasqué Strasse 35 A**
**D-6146 Alsbach(DE)**

Haarbehandlungsmittel mit einem Gehalt an D,L-Mandelsäure.

Ein Haarbehandlungsmittel, im wesentlichen frei von Konservierungsstoffen, mit einem pH-Wert von 2,5 bis 6,8, welches 0,02 bis 2,0 Gewichtsprozent D,L-Mandelsäure enthält, sowie die Verwendung von D,L-Mandelsäure zur Konservierung von Haarbehandlungsmitteln. Durch den Gehalt an D,L-Mandelsäure wird das Haarbehandlungsmittel bei einer sehr guten physiologischen Verträglichkeit wirksam konserviert.

EP 0 272 438 A1

## Haarbehandlungsmittel mit einem Gehalt an D,L-Mandelsäure

Gegenstand der Erfindung ist ein Haarbehandlungsmittel mit einem Gehalt an D,L-Mandelsäure sowie deren Verwendung als Konservierungsstoff in Haarbehandlungsmitteln.

Haarbehandlungsmittel enthalten in der Regel einen Konservierungsstoff, welcher die Mittel vor dem Befall durch Mikroorganismen wirksam schützen soll.

Für den Einsatz in Haarbehandlungsmitteln werden an einen Konservierungsstoff verschiedene Anforderungen gestellt. So sollte er einerseits in physiologischer und dermatologischer Hinsicht gut verträglich sein und andererseits über eine gute keimhemmende oder sogar keimtötende Wirkung verfügen. Beide Anforderungen sind meist nur schwer miteinander vereinbar.

Als übliche in Haarbehandlungsmitteln verwendete Konservierungsstoffe seien beispielsweise Formaldehyd, 5-Brom-5-nitro-1.3-dioxan, p-Hydroxybenzoesäureester, 2,4,4'-Trichlor-2'-hydroxy-diphenylether, 5-Chlor-2-methyl-3-isothiazolon, 2-Methyl-3-isothiazolon und 2-Brom-2-nitropropan-1,3-diol genannt.

In letzter Zeit sind einige Konservierungsstoffe, wie beispielsweise Formaldehyd und 2-Methyl-3-isothiazolon, in den Verdacht geraten, nicht genügend verträglich zu sein. Von konservierend wirkenden Aldehyden und Phenolen ist bekannt, daß diese mit Proteinen reagieren oder mit diesen in denaturierender Weise in Wechselwirkung treten. Bei Aldehyden besteht zudem eine große Gefahr der Sensibilisierung, während Phenole, wie zum Beispiel die Salicylsäure, insbesondere in höheren Konzentrationen, keratolytisch wirken.

Aus den vorstehend genannten Gründen werden die Aldehyde, soweit möglich, als Konservierungsstoffe umgangen und die Phenole in möglichst niedriger Konzentration in Haarbehandlungsmitteln eingesetzt.

Es ist offensichtlich, daß ein Bedarf an einem Konservierungsstoff für Haarbehandlungsmittel besteht, welcher weder zur Gruppe der Aldehyde noch der Phenole gehört, und welcher eine gute konservierende Wirkung sowie eine gute Verträglichkeit aufweisen soll.

Hierzu wurde nun gefunden, daß durch die Verwendung von D,L-Mandelsäure als Konservierungsstoff in Haarbehandlungsmitteln die gestellten Anforderungen in vollem Umfang erfüllt werden.

Die D,L-Mandelsäure ist nicht sensibilisierend, nicht mutagen und gut hautverträglich. Weiterhin besitzt sie eine sehr gute keimhemmende und keimtötende Wirkung. Die Verwendung von D,L-Mandelsäure als Konservierungsstoff wird erheblich dadurch erleichtert, daß sie sehr gut in Wasser löslich und völlig geruchsneutral ist. Darüberhinaus ist D,L-Mandelsäure in sehr hoher Reinheit und preisgünstig im Handel erhältlich.

Gegenstand dieser Anmeldung ist somit ein Haarbehandlungsmittel, welches im wesentlichen frei von Konservierungsstoffen ist und einen pH-Wert von 2.5 bis 6.8 besitzt, dadurch gekennzeichnet, daß es 0,02 bis 2,0 Gewichtsprozent, vorzugsweise 0,15 bis 0,8 Gewichtsprozent, D,L-Mandelsäure enthält.

Obwohl die Mandelsäure in den Haarbehandlungsmitteln vorzugsweise als alleiniger Konservierungsstoff eingesetzt wird, können zusätzlich bis zu 0,15 Gewichtsprozent eines oder mehrerer bekannter Konservierungsstoffe, beispielsweise p-Hydroxybenzoesäuremethylester oder Salicylsäure, eingesetzt werden, falls die in dem Mittel enthaltenen Rohstoffe sehr stark von Mikroorganismen befallen sind.

Die in der Anmeldung beschriebenen Haarbehandlungsmittel können in einer beliebigen für die Haarbehandlung geeigneten Zubereitungsform, wie zum Beispiel in Form einer Lösung, insbesondere einer wäßrigen oder wäßrig-alkoholischen Lösung, einer Lotion, einer Creme, einer Emulsion, eines Gels oder auch in Form eines Aerosols vorliegen. Bevorzugte Zubereitungen sind Haarspülungen, Haarpflegeemulsionen, Haarkurpackungen, Haarfestiger oder Shampoos. Die erfindungsgemäßen Haarbehandlungsmittel können aber auch als Haarfärbemittel, Haartönungsmittel oder als Fixiermittel für die Haarverformung vorliegen.

Es handelt sich dabei um Zubereitungen, die je nach ihrem Anwendungszweck für kürzere oder längere Zeit auf dem Haar verbleiben.

Die Zusammensetzung dieser haarkosmetischen Zubereitungen stellt eine Mischung der konservierend wirkenden D,L-Mandelsäure mit weiteren für das jeweilige Haarbehandlungsmittel erforderlichen Wirkstoffen, wie zum Beispiel Haarfarbstoffen oder kosmetischen Harzen, und üblichen Bestandteilen dar.

Als übliche Bestandteile von Haarbehandlungsmitteln kommen insbesondere Wasser, niedere aliphatische Alkohole, wie zum Beispiel Ethanol, Propanol und Isopropanol, mehrwertige Alkohole, wie Glycerin und Propylenglykol, kationische, anionische, amphotere oder nichtionogene Tenside, wie zum Beispiel Fettalkoholsulfate, Fettalkoholethersulfate, Alkylsulfonate, Alkylbenzolsulfonate, Fettsäuretauride, oxethylierte Fettalkohole, oxethylierte Alkylphenole, Fettsäurealkanolamide, Alkyltrimethylammoniumsalze, Alkylbetaine, weiterhin natürliche, modifizierte natürliche oder synthetische Polymere, wie zum Beispiel Schellack, Alginate,

2

Gelatine, Pektine, Cellulosederivate, Chitosan, Polyvinylpyrrolidon, Polyvinylacetat, Acrylsäure-oder Methacrylsäurepolymerisate, basische Polymerisate von Estern aus Acrylsäure oder Methacrylsäure mit Aminoalkoholen beziehungsweise die Salze oder Quaternisierungsprodukte dieser basischen Polymerisate, Polyacrylnitril sowie Copolymerisate aus derartigen Verbindungen, wie beispielsweise Polyvinylpyrrolidon-Vinylacetat, ferner Verdicker, wie zum Beispiel natürliche Öle, Stärke, Paraffinöl, Wachse, Vaseline und höhere Fettalkohole, weiterhin physiologisch verträgliche anorganische Salze, wie zum Beispiel Natriumchlorid, sowie außerdem Pflegestoffe wie zum Beispiel Lanolinderivate, Cholesterin und Pantothensäure, weiterhin Farbstoffe, Pigmente, Antischuppenwirkstoffe, Komplexbildner, Parfümöle, Treibgase und Antioxidantien in Betracht.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,1 bis 25 Gewichtsprozent und die Pflegestoffe in einer Menge von etwa 0,1 bis 5 Gewichtsprozent in den Zubereitungen enthalten sein können.

Die nachfolgenden Beispiele sollen den Gegenstand der Erfindung näher erläutern ohne ihn darauf zu beschränken.

**Beispiele für Haarbehandlungsmittel**

**Beispiel 1:** Haarwaschmittel

```
    0,6 g  DL-Mandelsäure
   40,0 g  Laurylalkohol-diglykolethersulfat, Natriumsalz
           (26prozentige wäßrige Lösung)
    0,5 g  Parfümöl
    4,0 g  Natriumchlorid
   54,9 g  Wasser
  _____

  100,0 g
```

Der pH-Wert wurde mit verdünnter Natronlauge auf 4,5 bis 5,5 eingestellt. Das Haarwaschmittel nach Beispiel 1 zeigt im Konservierungsbelastungstest eine sehr gute Konservierung.

3

**Beispiel 2:** Haarwaschmittel

```
      0,6 g DL-Mandelsaure
     16,0 g Laurylalkohol-diglykolethersulfat, Natriumsalz
            (70prozentige wäßrige Lösung)
      4,0 g Natriumchlorid
     79,4 g Wasser
     ─────────
    100,0 g
```

Der pH-Wert wird mit verdünnter Natronlauge auf 4.5 bis 5.5 eingestellt.

Das Haarwaschmittel nach Beispiel 2 ist trotz der fehlenden Vorkonservierung des darin enthaltenen Waschrohstoffs sehr gut konserviert.

**Beispiel 3:** Haarfestiger

```
      8,0 g DL-Mandelsäure (10prozentige wäßrige Lösung)
      1,0 g Chitosan, niedrigviskos (zu 80 Prozent entacety-
            liert)
      7,0 g Ethanol
     84,0 g Wasser
     ─────────
    100,0 g
```

In dem Haarfestiger nach Beispiel 3 dient DL-Mandelsäure sowohl zur Auflösung (Salzbildung) von Chitosan als auch zur Konservierung. Der pH-Wert der Lösung ergibt sich zu 3.6 bis 4.0. Die Konservierung ist sehr gut.

4

**Beispiel 4:** Haarkurmittel - Rinse

```
    0,30  g  DL-Mandelsäure
    1,00  g  Stearylalkohol
    1,00  g  Vaseline
    0,25  g  Glycerinmonostearat
    0,25  g  Glycerindistearat
    0,25  g  Cetyl-trimethyl-ammoniumchlorid
   96,95  g  Wasser
         _____

  100,00  g
```

Das Kurmittel nach Beispiel 4 mit einem pH-Wert von 2,5 bis 3,5 ist sehr gut konserviert.

**Beispiel 5:** Haartönungsmittel

```
    0,15  g  DL-Mandelsäure
    2,00  g  Cetylstearylalkohol
    0,40  g  Natriumlaurylsulfat
    0,10  g  Disperse Blue 3
    0,05  g  2-Amino-4-nitro-phenol
    0,05  g  p-Hydroxy-benzoesäuremethylester
   97,25  g  Wasser
         _____

  100,00  g
```

Das Haartönungsmittel nach Beispiel 5 mit einem pH-Wert von 4 bis 5 ist gut konserviert.

Die Prozentangaben dieser Anmeldung stellen, soweit nicht anders vermerkt, Gewichtsprozente dar.

Die Güte der Konservierung der vorstehend beschriebenen Haarbehandlungsmittel wurde durch den in der Literatur The United States Pharmacopeia, 21. Auflage, (1985), Seite 1151 beschriebenen Konservierungsbelastungstest festgestellt. Hierbei wurden die Haarbehandlungsmittel mit den Mikroorganismen

Candida albicans (ATCC Nr. 10231), Aspergillus niger (ATCC Nr. 16404), Escherichia coli (ATCC Nr. 8739), Pseudomonas aeruginosa (ATCC Nr. 9027), and Staphylococcus aureus (ATCC Nr. 6538)

verunreinigt und die Entwicklung der Keimzahl über einen Zeitraum von 28 Tagen überwacht.

## Ansprüche

1. Haarbehandlungsmittel, im wesentlichen frei von Konservierungsstoffen, mit einem pH-Wert von 2,5 bis 6,8, dadurch gekennzeichnet, daß es 0,02 bis 2,0 Gewichtsprozent D,L-Mandelsäure enthält.

2. Mittel nach Anspruch 1, dadurch gekennzeichnet, daß es 0,15 bis 0,8 Gewichtsprozent D,L-Mandelsäure enthält.

3. Mittel nach den Ansprüchen 1 und 2. dadurch gekennzeichnet. daß es als Lotion. als Gel. als Creme oder als Aerosol vorliegt.

4. Mittel nach den Ansprüchen 1 bis 3. dadurch gekennzeichnet. daß es niedere aliphalische Alkohole. kationische. anionische. amphotere oder nichtionogene Tenside. natürliche. modifizierte natürliche oder synthetische Polymere. Verdicker. Pflegestoffe. Farbstoffe. Pigmente. Parfümöle. Komplexbildner oder Antioxidantien enthält.

5. Verwendung von D.L-Mandelsäure zur Konservierung von Haarbehandlungsmitteln mit einem pH-Wert von 2.5 bis 6.8.

6

| | | |
|---|---|---|
| **Europäisches Patentamt** | **EUROPÄISCHER RECHERCHENBERICHT** | Nummer der Anmeldung |
| | | EP 87 11 6700 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 105 782 (R.J. YU et al.)<br>* Insgesamt *<br>--- | 1,3,4 | A 61 K 7/06<br>A 61 K 7/00 |
| X | CHEMICAL ABSTRACTS, Band 58, Spalte 5456a, Columbus, Ohio, US; F. MELSON et al.: Effect of bacterial infection of Lanette ointment by various bactericidal substances", & PHARMAZIE 17, 614-16(1962)<br>* Zusammenfassung *<br>--- | 5 | |
| A | US-A-3 988 363 (SPIVACK et al.)<br>* Insgesamt *<br>--- | 1-5 | |
| A | CHEMICAL ABSTRACTS, Band 37, Spalte 3123, Punkt 5, Columbus, Ohio, US;G.P. HAGER et al.: " A study of the bacteriostatic activity of fluoro and bromo derivatives of some organic acids", & J. INFECTIOUS DISEASES 71, 228-31(1942)<br>* Zusammenfassung *<br>----- | 5 | |

**RECHERCHIERTE SACHGEBIETE (Int. Cl.4)**

A 61 K

Der vorliegende Recherchenbericht wurde für alle Patentansprüche erstellt

| Recherchenort | Abschlußdatum der Recherche | Prüfer |
|---|---|---|
| DEN HAAG | 03-02-1988 | FISCHER J.P. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsatze
E : alteres Patentdokument, das jedoch erst am oder
    nach dem Anmeldedatum veröffentlicht worden ist
D : in der Anmeldung angeführtes Dokument
L : aus andern Grunden angeführtes Dokument
.......................................................................
& : Mitglied der gleichen Patentfamilie, ubereinstimmendes
    Dokument

EPO FORM 1503 03 82 (P0403)